(19) Europäisches Patentamt
European Patent Office
Office européen des brevets

(11) Publication number: **0 446 185 A1**

# (12) EUROPEAN PATENT APPLICATION

(21) Application number: **91830070.8**

(51) Int. Cl.5: **C07C 323/58**, A61K 31/22

(22) Date of filing: **04.03.91**

(30) Priority: **06.03.90 IT 4772490**

(43) Date of publication of application:
**11.09.91 Bulletin 91/37**

(84) Designated Contracting States:
**AT BE CH DE DK ES FR GB GR IT LI LU NL SE**

(71) Applicant: **SIGMA TAU Industrie Farmaceutiche Riunite S.p.A.**
**Viale Shakespeare, 47**
**I-00144 Rome(IT)**

(72) Inventor: **Tinti, Maria Ornella**
**81, Via Ernesto Basile**
**I-00182 Rome(IT)**
Inventor: **Misiti, Domenico**
**3, Via Bacchiglione**
**I-00199 Rome(IT)**
Inventor: **Cavazza, Claudio**
**2, Piazza Campitelli**
**I-00186 Rome(IT)**

(74) Representative: **Fassi, Aldo**
**c/o Sigma-Tau Industrie Farmaceutiche Riunite S.p.A., Viale Shakespeare 47**
**I-00144 Rome(IT)**

## (54) Alpha-amino-omega-alkylthioacylcarnitines active on the cardiovascular system and pharmaceutical compositions containing same.

(57) Alpha-amino-omega-alkylthioacylcarnitines, particularly L-methionyl L-carnitine, are drugs active on the cardiovascular system, particularly for the treatment of myocardial anoxia, cardiac ischaemia, arrhythmias and congestive heart failure.

EP 0 446 185 A1

The present invention relates to alpha-amino-omega-alkylthioacylcarnitines, particularly L-methionyl L-carnitine and the pharmacologically acceptable salts thereof.

These compounds are active on the cardiovascular system. For the sake of simplicity, hereinbelow we will make reference to L-methionyl L-carnitine, this compound having formula

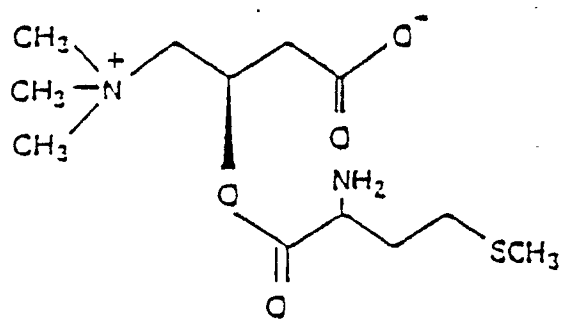

(I)

It is apparent that the pharmacologically acceptable salts of the compound of formula (I) can either have general formula (I')

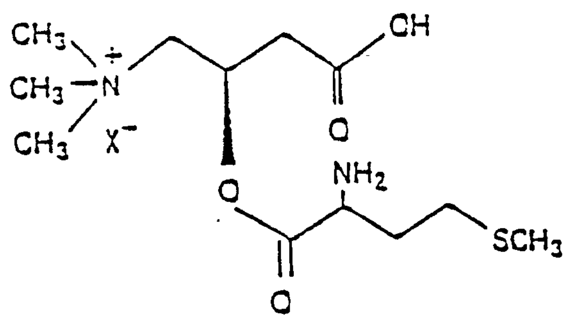

(I')

wherein $X^-$ is the anion of a pharmacologically acceptable acid e.g. chloride, bromide, orotate, acid aspartate, acid citrate, acid phosphate, acid fumarate, lactate, acid maleate, acid oxalate, acid sulfate and glucosephosphate; or they have general formula (I'')

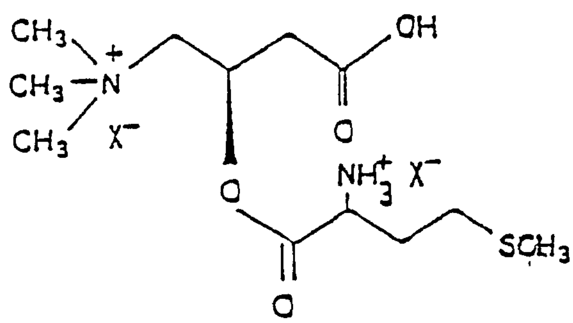

(I'')

wherein $X^-$ has the previously defined meaning.

Since salts (I'') are more stable than both salts (I') and inner salts (I) and since in any case the conversion from salts (I'') or (I') to inner salts is conducted via known procedures (using e.g. ion exchange resins the proper choice of which is within the reach of anyone skilled in the art), for the sake of simplicity, the specification that follows deals with the salts of formula (I'').

L-amino acyl derivatives of L-carnitine are already known:

cfr. Italian patent 1 208 753 or its corresponding EP publication 252030 or its corresponding US patent 4,812,478. These known compounds, that have formula

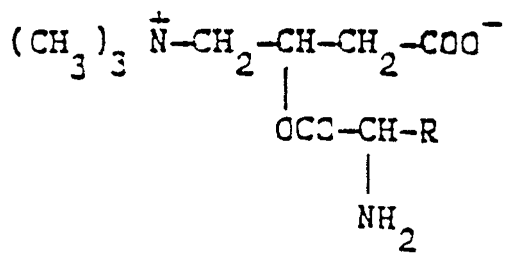

wherein R is an alkyl radical selected from 1 -methylpropyl, isobutyl, isopropyl, mercaptomethyl and 3-guanidinopropyl, and their pharmacologically acceptable salts, are useful as hepato protective drugs.

The compounds of the present invention are totally remote from the known compounds, not only from a structural viewpoint because of the presence of the alkylthio group (particularly, methylthio in the compounds (I), (I') and (I")), but also for their totally unrelated therapeutic utilisation. The compounds of the present invention are, in fact, active on the cardiovascular system, particularly for the treatment of myocardial anoxia, cardiac ischaemia, arrhythmias and congestive heart failure.

The following non-limiting example describes the preparation of L-methionyl L-carnitine chloride hydrochloride (ST 668).

**Example 1** Preparation of L-methionyl L-carnitine chloride hydrochloride (ST 668)

Preparation of N-carbobenzoxy L-methionyl L-carnitine benzylester perchlorate.
N-carbobenzoxy L-methionine (56 g; 0.2 moles) was dissolved in 200 cc anhydrous tetrahydrofurane.
N,N'-carbonyldiimidazole (36.4 g; 0.224 moles) was added to the solution at 0 °C, under a nitrogen atmosphere. The resulting solution was kept under stirring at room temperature for 5 hours.
Dimethylaminopyridine (2.4 g; 0.002 moles) and a solution of L-carnitine benzylester perchlorate (70 g; 0.2 moles) in 100 cc anhydrous tetrahydrofurane were then added. The resulting mixture was kept under stirring at room temperature for 48 hours.
The raw reaction product was concentrated under vacuum, the residue thus obtained was dissolved in $CH_2Cl_2$ and washed three times with 2% HCl and again three times with $H_2O$.
The organic phase, dried over anhydrous $Na_2SO_4$, was concentrated under vacuum.
100 g of a hygroscopic solid product were obtained. Yield 80%.
The product was analyzed via HPLC.

| | |
|---|---|
| Column | Lichrosorb RP - 8 |
| Eluant | $KH_2PO_4$ 0.05 M -$CH_3CN$ 40 - 60 |
| Pressure | 57 atm. (58.881 Kg/cm$^2$) |
| Flow rate | 1 ml/min |
| Detector | UV $\lambda$ 205 nm |
| Chart speed | 0.5 cm/min |
| RT | 7.4 |

Preparation of N-carbobenzoxy L-methionyl L-carnitine benzylester chloride.
A methanol solution of N-carbobenzoxy L-methionyl L-carnitine benzylester perchlorate (100 g; 0.16 moles) was loaded on a column of 100 ml AMBERLIST
A 21 resin activated in $Cl^-$ form.
The resin was eluted with methanol.
The concentrated methanol solution gave 80 g of a hygroscopic, white product (yield 70%).
The product thus obtained was purified via preparative HPLC:

| | |
|---|---|
| Waters | Delta Prep 3000 |
| Column | Prepak $C_{18}$ a 750 PSI |
| Eluant | $CH_3CN$ 50: $H_2O$ 50 |
| Flow rate | 20 ml/min |

80 g of product were dissolved in 800 ml $CH_3CN$, filtered through a W 42 filter and injected in the chromatograph. The collected fractions were concentrated under vacuum and analyzed via HPLC under the same conditions previously described.

The fractions having $R_t = 13.8$ were joined furnishing 34 g of pure product, yield 31%.

Conversion of N-carbobenzoxy L-methionyl L-carnitine benzylester chloride to ST 668.
N-carbobenzoxy L-methionyl L-carnitine benzylester chloride (46 g; 0.083 moles) was dissolved in 200 cc $H_2O$.
7 cc concentrate HCl were added to the solution.
The resulting solution was hydrogenated in the presence of 10 g 10% Pd/C under pressure (40 psi; 2.8 $Kg/cm^2$) at room temperature, for 24 hours.
To the filtered solution 10 g 10% Pd/C were further added and the hydrogenation continued for 24 hours.
The solution was filtered and lyophilized.
30 g of a hygroscopic white product were obtained. The product was purified via preparative HPLC:

| | |
|---|---|
| Chromatograph | Water Delta prep 3000 |
| Column | Prepack $C_{18}$ |
| Eluant | 100% $H_2O$ |
| Flow rate | 20 ml/min |
| Column pressure | 750 PSI (52.7 $kg/cm^2$) |
| Eluant pressure | 40 PSI (2.8 $kg/cm^2$) |

A solution of 30 g of the product dissolved in 600 ml $H_2O$ was filtered through a W 42 filter and injected in the chromatograph. The collected fractions immediately frozen, were lyophilized. 15 g of a hygroscopic, white product were obtained. Yield 50%
$[\alpha]_D^{25} = -7.1$ (C = 1 $H_2O$)

T.L.C. = $CHCl_3, CH_3OH, H_2O$, IPR-OH, $CH_3COOH$
60 40 15 10 15

Rf = 0.3 detector ninidrine
M.P. = 120° C dec. (68° C soft)
HPLC
Column μ Bondapack $NH_2$
Eluant:

| | |
|---|---|
| $KH_2PO_4$ 0.05M | 35 |
| $CH_3CN$ | 65 |

Flow rate 1 ml/min
Detector: UV λ 205 nm
Rt = 14.5

NMR $D_2O$ δ

5.6 (1H, m-CH-[O-]); 4.3 (1H, t, -CH-[$NH_2$]);

3.8 (2H, m, $N^+$ -$CH_2$-); 3.2 (3H, s, $(CH_3)_3N^+$); 2.8 (2H, d, $CH_2COOH$-); 2.5 (2H, m, $CH_2S$); 2.3-2.0 (5H, m + s, $CH_2$-$CH_2SCH_3$)

The compounds of the present invention are orally or parenterally administered, in any of the usual pharmaceutical forms which are prepared by conventional procedures well-known to those persons skilled in the pharmaceutical technology. These forms include solid and liquid oral unit dosage forms such as tablets, capsules, solution, syrups and the like as well as injectable forms, such as sterile solutions for ampoules and phials.

For these pharmaceutical forms the usual solvents, diluents and excipients are used. Optionally, sweetening, flavouring and preservative agents can also be present. Non limiting examples of such agents are sodium carboxymethylcellulose, polysorbate, mannitol, sorbitol, starch, avicel, talcum and other agents

which will be apparent to those skilled in the pharmaceutical technology.

The dose which is administered will be determined by the attending physician having regard to the age, weight and general conditions of the patient, utilizing sound professional judgement. Although effective results can be noticed at doses as low as 5 to 8 mg/kg of body weight daily, a dose from about 10 to about 50 mg/kg of body weight is preferred. Whenever necessary, larger doses can be safely administered in view of the low toxicity of the compounds of this invention.

As non-limiting examples and depending on the specific pharmaceutical form of administration, the following dosages can be indicated:

| | |
|---|---|
| for the phials | : from 5 to 500 mg |
| for the capsules | :from 15 to 50 mg |
| for the tablets | :from 15 to 500 mg |
| for the oral solution | :from 15 to 50 mg. |

## Claims

1. Alpha-amino-omega-alkylthioacylcarnitines and their pharmacologically acceptable salts.

2. L-methionyl L-carnitine and its pharmacologically acceptable salts.

3. The compound of claim 2, as inner salt of formula (I)

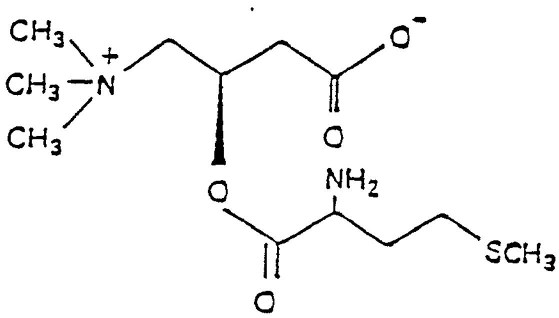

(I)

4. The compound of claim 2, as pharmacologically acceptable salt of formula (I')

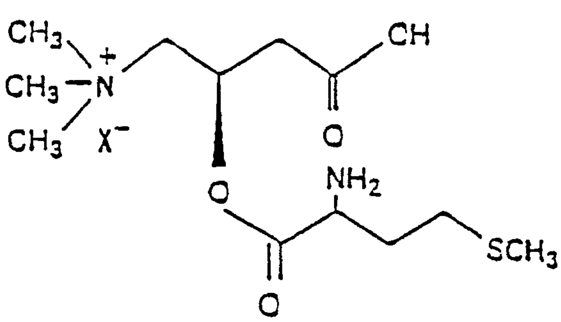

(I')

wherein $X^-$ is the anion of a pharmacologically acceptable acid.

5. The compound of claim 2, as pharmacologically acceptable salt of formula (I")

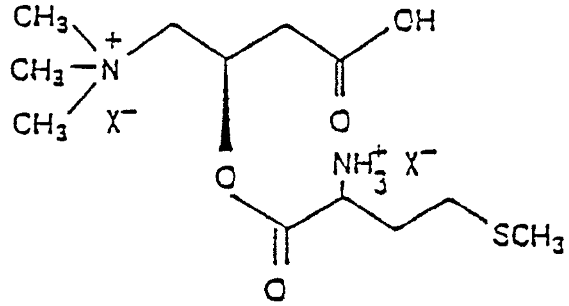

(I'')

wherein $X^-$ is the anion of a pharmacologically acceptable acid.

6. The compounds of claims 4 or 5, wherein $X^-$ is selected from chloride, bromide, orotate, acid aspartate, acid citrate, acid phosphate, acid fumarate, lactate, acid maleate, acid oxalate, acid sulfate and glucosephosphate.

7. An orally or parenterally administrable pharmaceutical composition comprising a compound of formula (I), (I') or (I'') as active principle.

8. An orally or parenterally administrable pharmaceutical composition for the therapeutical treatment of myocardial anoxia, cardiac ischaemia, arrhythmias and congestive heart failure, comprising a compound of formula (I), (I') or (I'') as active principle and a pharmacologically acceptable excipient therefor.

9. The composition of claim 8 in unit dosage form, comprising from about 5 to about 500 mg of a compound of formula (I), (I') or (I'').

**Claim for the following Contracting States: ES, GR**

1. A process for producing L-methionyl L-carnitine chloride hydrochloride which comprises the following steps:

(a) reacting at 0° C - 30° C for 0.5 - 24 hours benzyl L-carnitine perchlorate in an inert anhydrous organic solvent selected from tetrahydrofurane and acetonitrile, with N-carbobenzoxy L-methionine, in the presence of N,N'-carbonyldiimidazole, thus obtaining N-carbobenzoxy L-methionyl L-carnitine benzyl ester perchlorate;

(b) substituting the chloride ion for the perchlorate anion by means of a weakly basic ion exchange resin activated in $Cl^-$ form by eluting the compound of step (a) in an alcoholic or aqueous-alcoholic solvent, thus obtaining N-carbobenzoxy L-methionyl L-carnitine benzylester chloride; and

(c) removing the N-carbobenzoxy group of the aminoacid and the benzyl radical of benzyl L-carnitine by hydrogenation in the presence of a hydrogenation catalyst selected from 10% Pd/C and $PtO_2$ in an alcoholic, aqueous-alcoholic solvent or water at room temperature at a pressure of 1 - 4 $kg/cm^2$.

European Patent Office

# EUROPEAN SEARCH REPORT

Application Number

**EP 91 83 0070**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 429 776 (SIGMA-TAU)<br>* Page 26, line 25; claim 5 *<br>- - - | 1-9 | C 07 C 323/58<br>A 61 K 31/22 |
| D,A | EP-A-0 252 030 (SIGMA-TAU)<br>- - - - - | | |
| | | | TECHNICAL FIELDS SEARCHED (Int. Cl.5) |
| | | | C 07 C 323/00 |

The present search report has been drawn up for all claims

| Place of search | Date of completion of search | Examiner |
|---|---|---|
| The Hague | 11 June 91 | ZAROKOSTAS K. |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document